# EUROPEAN PATENT APPLICATION

(11) **EP 4 763 056 A1**
(43) Date of publication of application: **24.06.2026**
(21) Application number: 25218972.5
(22) Date of filing: 27.11.2025
(51) Int. Cl.: A61B 5/00, A61B 5/344, A61B 5/288, A61B 5/391, A61B 5/024

(54) **DETERMINING FETAL PARAMETERS USING AN ELECTRODE GRID PATCH ON THE MATERNAL ABDOMEN**

(30) Priority: 18.12.2024 US 202418985709
(71) Applicant: GE Precision Healthcare LLC, Waukesha, WI 53188 (US)
(72) Inventor: MANICKAM, Kalaivani, Waukesha, 53188 (US); KAVOORI SETHUMADHAVAN, Nagapriya, Waukesha, 53188 (US); NAIK, Rajendra, Waukesha, 53188 (US)
(74) Representative: Kilburn & Strode LLP

(57) **Abstract**

A method includes determining fetal parameters using at least one electrode grid patch that is disposed on a maternal abdomen of a pregnant patient, by receiving an abdominal electromyogram (EMG) signal from the at least one electrode grid patch. Additionally, the at least one electrode grid patch includes multiple electrodes that are disposed in at least one array. Further, the method includes determining fetal parameters by determining for a fetus of the pregnant patient, based on the EMG signal, a fetal presentation. Further, the method includes providing for display on a display device, the fetal presentation.

## Description

### BACKGROUND

The present disclosure generally relates to an electrode grid patch, and more particularly to determining fetal parameters using an electrode grid patch for the maternal abdomen.

In clinical applications, it is useful to apply electrode patches to the surface of the patient's skin to monitor aspects of the patient's health by measuring specific physiological conditions. These patches include surface electrodes, which, when adhered to the surface of the patient's skin, enable electrical contact between the patient's skin and a conductor. Additionally, the conductor may connect the surface electrodes to a sensor that measures the physiological condition. In some cases, the surface electrodes may conduct potentials from the patient's body, e.g., the electric potential from contracting muscle, thus enabling their measurement. This physiological monitoring may be useful for tracking muscle contractions, temperature, respiration rates, pulse rates, and the like. In this way, the electrode patches may provide data that is useful for producing electrocardiograms (ECGs), electroencephalograms (EEGs), and the like.

### SUMMARY

This Summary is provided to introduce a selection of concepts that are further described below in the Detailed Description. This Summary is not intended to identify key or essential features of the claimed subject matter, nor is it intended to be used as an aid in limiting the scope of the claimed subject matter.

A method includes determining fetal parameters using at least one electrode grid patch that is disposed on a maternal abdomen of a pregnant patient, by receiving an abdominal electromyogram (EMG) signal from the at least one electrode grid patch. Additionally, the at least one electrode grid patch includes multiple electrodes that are disposed in at least one array. Further, the method includes determining fetal parameters by determining for a fetus of the pregnant patient, based on the EMG signal, a fetal presentation. Further, the method includes providing for display on a display device, the fetal presentation.

In one embodiment, the method includes extracting, from the EMG signal: a maternal ECG signal and a uterine activity signal. Additionally, the method includes determining for the fetus, based on the EMG signal: a fetal heart location, a fetal position, and a fetal station.

In another embodiment, determining the fetal presentation, the fetal position and the fetal heart location includes performing a vectorial analysis on the EMG signal.

In another embodiment, determining the fetal presentation, the fetal position, and the fetal heart location includes performing a vectorial analysis on the fetal ECG signal.

In another embodiment, the method includes generating amplitude and direction of the fetal ECG signal by performing vectorial analysis.

In another embodiment, the method includes performing vectorial analysis on the EMG signal. Additionally, performing the vectorial analysis includes generating multiple vectors corresponding to the electrodes. Further, each of the vectors includes an amplitude and a direction for the components of the EMG signal. Additionally, performing the vectorial analysis includes determining multiple resultant vectors corresponding to the vectors. Further, each of the resultant vectors results from a comparison parameter between the amplitude and the direction for each of the vectors for each of the electrodes. Additionally, performing the vectorial analysis includes determining the fetal heart location, the fetal presentation and the fetal position based on the resultant vectors.

In another embodiment, determining the fetal presentation includes performing a pattern recognition analysis of the fetal ECG signal

In another embodiment, determining the fetal presentation, the fetal heart location, and the fetal position includes performing a classification on a sequential time-series analysis of the EMG signal.

In another embodiment, determining the fetal station, the fetal presentation, the fetal position, and the fetal heart location include generating multiple sequential input mapping images of the maternal abdomen based on the EMG signal. Additionally, determining the station, presentation, and heart location include using a sequential deep learning model that is trained to classify the fetal station, the fetal presentation, the fetal position, and fetal heart location, based on the plurality of input mapping images.

In another embodiment, determining the fetal ECG signal, maternal ECG and uterine activity signal include increasing signal quality and reducing signal noise by performing multi-electrode analysis and signal processing techniques on the EMG signal.

In another embodiment, the method includes extracting, from the EMG signal an additional fetal ECG signal for an additional fetus, and determining for the additional fetus, based on the EMG signal, an additional fetal presentation, an additional fetal heart location, an additional fetal position, and an additional fetal station.

In another embodiment, extracting the additional fetal ECG signal includes separating the fetal ECG signal from the additional fetal ECG signal by using source separation methods.

A system includes at least one electrode grid patch, a processing device, and a memory. The electrode grid patch includes multiple electrodes disposed in at least one array. Additionally, the electrode grid patch is configured for placement on a maternal abdomen of a pregnant patient, and to conduct potentials of a pregnant patient, and a fetus of the pregnant patient. The memory includes instructions that are executable by the processing device to receive an EMG signal from at least one electrode grid patch, the at least one electrode grid patch including multiple electrodes that are disposed in at least one array. Further, the instructions are executable by the processing device to determine for the fetus, based on the EMG signal, a fetal position. Additionally, the instructions are executable by the processing device to provide for display on a display device, the fetal position.

In one embodiment, the instructions are executable by the processing device to extract, from the EMG signal: a maternal ECG signal and a uterine activity signal. Further, the instructions are executable by the processing device to determine for the fetus, based on the EMG signal: a fetal heart location, a fetal presentation, and a fetal station.

In another embodiment, the fetal position, the fetal presentation, and the fetal heart location are determined by performing a vectorial analysis on the fetal ECG signal, the maternal ECG signal, and the uterine activity signal.

In another embodiment, the fetal position, the fetal presentation, and the fetal heart location are determined by performing a vectorial analysis on the EMG signal.

In another embodiment, the instructions are executable by the processing device to perform the vectorial analysis by generating multiple vectors corresponding to the electrodes. Further, each of the vectors includes an amplitude and a direction for multiple components of the EMG signal. Additionally, the vectorial analysis is performed by determining multiple resultant vectors corresponding to the vectors. Further, each of the resultant vectors results from a comparison parameter between the amplitude and the direction for each of the vectors for each pair of the electrodes. Additionally, the vectorial analysis is performed by determining the fetal heart location, the fetal presentation and the fetal position based on the resultant vectors.

In another embodiment, the instructions are executable by the processing device to determine a fetal engagement of the fetus based on the fetal ECG signal, the maternal ECG signal, and the uterine activity signal.

In another embodiment, determining the fetal presentation, the fetal heart location, and the fetal engagement include using a sequential artificial intelligence model that is trained to infer the fetal presentation, the fetal heart location, and the fetal engagement, at a plurality of times, based on the EMG signal.

A method includes determining fetal parameters using at least one electrode grid patch that is disposed on a maternal abdomen of a pregnant patient, by receiving an abdominal electromyogram (EMG) signal from the at least one electrode grid patch. Additionally, the at least one electrode grid patch includes multiple electrodes that are disposed in at least one array. Further, the method includes determining fetal parameters by determining for a fetus of the pregnant patient, based on the EMG signal, a fetal station. Further, the method includes providing for display on a display device, the fetal station.

Various other features, objects, and advantages of the invention will be made apparent from the following description taken together with the drawings.

### BRIEF DESCRIPTION OF THE DRAWINGS

The present disclosure is described with reference to the following Figures.
Fig. 1 is a side view of an example electrode grid patch on a maternal abdomen, according to one embodiment of the present disclosure.
Fig. 2A is a front view of the presentation of a fetus in a maternal abdomen, according to one embodiment of the present disclosure.
Fig. 2B is a front view of the presentation of a fetus in a maternal abdomen, according to one embodiment of the present disclosure.
Fig. 2C is a front view of the presentation of a fetus in a maternal abdomen, according to one embodiment of the present disclosure.
Fig. 2D is a front view of the presentation of a fetus in a maternal abdomen, according to one embodiment of the present disclosure.
Fig. 3A is a front view of the position of a fetus in a maternal abdomen, according to one embodiment of the present disclosure.
Fig. 3B is a front view of the position of a fetus in a maternal abdomen, according to one embodiment of the present disclosure.
Fig. 3C is a front view of the position of a fetus in a maternal abdomen, according to one embodiment of the present disclosure.
Fig. 3D is a front view of the position of a fetus in a maternal abdomen, according to one embodiment of the present disclosure.
Fig. 4A is a front view of an example electrode grid patch on a maternal abdomen, according to one embodiment of the present disclosure.
Fig. 4B is a front view of example electrode grid patches on maternal abdomen, according to one embodiment of the present disclosure.
Fig. 4C is a front view of example electrode grid patches on a maternal abdomen, according to one embodiment of the present disclosure.
Fig. 5 is an example time-series view of physiological monitoring from one of the electrodes of an example electrode grid patch, according to one embodiment of the present disclosure.
Fig. 6 is a diagram of an example electrode grid patch and a timeline of mapping images, according to one embodiment of the present disclosure.
Fig. 7 is a data flow diagram of a process for physiological monitoring using an example electrode grid patch, according to one embodiment of the present disclosure.
Fig. 8 is a diagram of an example electrode grid patch for maternal abdomen manager, according to one embodiment of the present disclosure.

### DETAILED DESCRIPTION

In the present description, certain terms have been used for brevity, clarity and understanding. No unnecessary limitations are to be inferred therefrom beyond the requirement of the prior art because such terms are used for descriptive purposes only and are intended to be broadly construed.

As used herein, unless otherwise limited or defined, discussion of particular directions is provided by example only, with regard to particular embodiments or relevant illustrations. For example, discussion of "top," "bottom," "front," "rear," "left," "right," "horizontal," "vertical," and "longitudinal" features and/or relative motion, e.g., movement "up" and "down," is generally intended as a description only of the orientation of such features relative to a reference frame of a particular example or illustration. Correspondingly, for example, a "top" feature may sometimes be disposed below a "bottom" feature (and so on), in some arrangements or embodiments. Additionally, or alternatively, embodiments may be arranged in a different orientation such that "top" and "bottom" features are arranged horizontally relative to each other, for example in a "left-to-right" orientation.

The use herein of the terms "including," "comprising," or "having," and variations thereof, is meant to encompass the elements listed thereafter and equivalents thereof, as well as additional elements. Embodiments recited as "including," "comprising," or "having" certain elements are also contemplated as "consisting essentially of" and "consisting of" those certain elements.

The inventors have recognized problems with current techniques for maternal and fetal monitoring. For example, to aid the mother in a safe labor and delivery, it is useful to know the position and presentation of the fetus with respect to the mother's body. The term, position, refers to the position of the fetal head as it exits the birth canal, and is described with respect to the orientation of the fetal occiput (i.e., the back of the skull). In many cases, the fetal head engages in the occipito-anterior position (the fetal occiput facing anteriorly, i.e., towards the maternal abdomen), which may facilitate a safe birth. However, other positions include occipito-posterior (i.e., facing towards the maternal abdomen), and occipito-transverse (i.e., facing sideways with respect to the maternal abdomen and spine). The presentation, however, indicates the position of the fetal head with respect to the pelvic inlet. For example, the presentation may be cephalic, breech, oblique, or transverse (which are described in greater detail below). Knowing the presentation may be useful in determining whether clinician should provide treatment, e.g., in a breech presentation. In current labor, delivery, and/or other clinical settings, a doctor and/or other clinician may determine the position and presentation of the baby by palpating the mother's abdomen, i.e., placing the hand(s) on the abdomen to feel the fetus within. As the fetus descends into the birth canal, the fetal descent is monitored during labor by assessing the fetal head station (i.e., engagement). Fetal station is monitored during labor and delivery using manual physical examination (a vaginal exam to feel the baby's head in relation to the ischial spines) or using an ultrasound.

Additionally, it may be useful to know the fetal heart rate, maternal heart rate, and other physiological measures. Accordingly, the clinician may measure the fetal heart rate (FHR) using ultrasound by positioning an ultrasound transducer on the abdomen within certain parameters, e.g., with respect to the distance and orientation between the transducer and the fetal heart. However, as the fetus descends, changes position and/or presentation, the transducer's location may fall outside the range of detectable distance for the fetal heart rate. Thus, the clinician may reposition the transducer for more effective fetal heart rate detection. However, such repositioning adds to the clinician's responsibilities in a potentially dangerous medical setting where the clinician's attention may already be otherwise fully occupied. Further, the change in fetal position may mean that the fetal heart rate goes undetected for some period of time. Such a scenario may raise the possibility that a change in fetal heart rate (possibly indicating medical intervention), may go undetected, potentially putting the mother's and baby's health at risk.

In view of the foregoing problems and challenges recognized by the inventors through their extensive research and experience in the field of maternal and fetal monitoring, the inventors have developed the disclosed method and system for maternal and fetal monitoring using electrode grid patch for the maternal abdomen. The abdominal electrode grid patch may provide physiological measures of the mother and fetus during pregnancy, and multiple stages of labor. Using these physiological measures, it may be possible to determine multiple aspects of the health of mother and baby, and the position, presentation, station, and heart location of the fetus.

Additionally, it may be possible to make these determinations despite the stage of labor, the number of fetuses, and/or the station of the fetus(es). In these ways, some embodiments of the present disclosure may provide an abdominal electrode grid patch that measures the fetal electrocardiogram (fECG), maternal ECG (mECG), and uterine activity (UA). Additionally, some embodiments of the present disclosure may determine the location of the fetal heart, the position and presentation of the fetus. Additionally, using the fetal location information, the fetal descent (station) can be tracked during labor and delivery. In these ways, some embodiments of the present disclosure may provide useful physiological information from one or more electrode grid patch that is securely positioned on the maternal abdomen, thus reducing the amount of work for the clinician during labor. Additionally, determining the fetal heart location, position, and/or presentation using such embodiments may reduce contact between the clinician and the mother, reduce discomfort for the mother, and reduce the clinician's labor because this information may be determined without palpation.

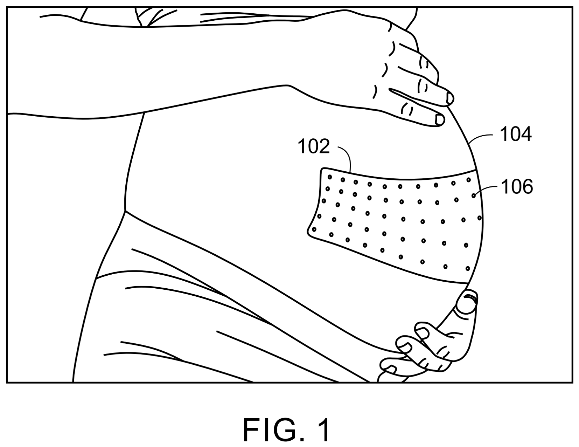

Fig. 1 is a side view of an example electrode grid patch 102 on a maternal abdomen 104 (i.e., the abdomen of a pregnant person), according to one embodiment of the present disclosure. The example electrode grid patch 102 is a high-density surface electromyogram (EMG) electrode patch for applying to the abdomen 104 of a pregnant woman, to detect the EMG signal, which may include fetal electrocardiogram (fECG), maternal ECG (mECG), and uterine electrical signals. According to some embodiments of the present disclosure, the example electrode grid patch 102 can detect the fetal position, presentation, heart location, and station.. In addition, as this system may also be used to determine fetal heart location and presentation, the example electrode grid patch 102 may be useful in positioning an ultrasound transducer to detect fetal heart rate. Further, the example electrode grid patch 102 may provide relatively high quality and relatively low noise fetal and maternal ECGs (and uterine activity signals). Further, such embodiments may reduce noise and increase signal extraction capabilities by combining signals across different electrodes or different pairs of grid electrodes 106, and using different signal processing methods (e.g., averaging) to remove/ reduce artifacts such as, movement. According to some embodiments of the present disclosure, all grid electrodes and/or electrode pairings may be compared and/or combined. Alternatively, some embodiments may use grid electrodes/ electrode pairings that lie in a predetermined direction. Additionally, some embodiments of the present disclosure may determine the grid electrodes/ pairings adaptively. For example, based on signal to noise levels and based on noise and artefact cancelations, electrodes are adaptively selected. Additionally, a spatial map of the muscle potentials may be obtained by measuring the potential difference between various electrode pairings or by using a reference electrode and measuring the potential difference between the each electrode and the reference electrode. Continuous measurement of the potentials can be useful for generating a spatiotemporal map, from which, a clinician may determine fetal heart location, fetal presentation and fetal station. More specifically, from the spatiotemporal map, the clinician may identify the properties of the muscles and/or muscle fibers (e.g., the locations of the innervation zones and the propagation properties, i.e., direction and muscle fiber conduction velocity), thereby making it possible to determine fetal heart location, fetal presentation and fetal station.

According to some embodiments of the present disclosure, the example electrode grid patch 102 may measure multi-channel EMG signals from the maternal abdomen with temporal and spatial information. More specifically, the example electrode grid patch 102 includes a grid of electrodes 106 (grid electrodes 106). As stated previously, electrodes, like those of the grid electrodes 106 conduct potentials beneath the skin of the abdomen 104.

In these ways, the example electrode grid patch 102 may make it possible to continuously detect fetal position, presentation, fetal heart location and station. Accordingly, such embodiments may eliminate the practice of periodic palpation of the maternal abdomen, which may be uncomfortable for the mother, and time-consuming for the clinician. Further, such embodiments make it possible to continuously determine fetal descent, and present it graphically on a monitor, smartphone, or other display. Additionally, such embodiments may be useful at home (where pre-term labor is more likely), and for high-risk pregnancies. Further, it may be possible to place the example electrode grid patch 102 in one position for monitoring throughout labor. In other words, such embodiments may eliminate the practice of changing the position of a patch throughout labor.

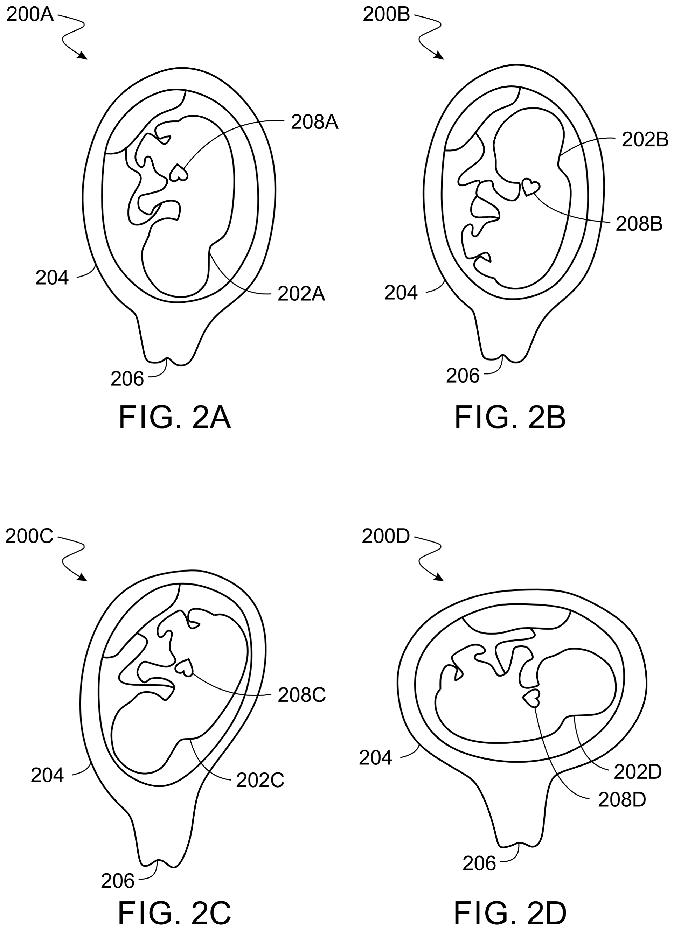

Figs. 2A, 2B, 2C, 2D are front views 200A, 200B, 200C, 200D (collectively referred to as views 200) of the respective presentations of fetuses 202A, 202B, 202C, 202D (collectively referred to as fetuses 202) in a maternal abdomen 204, according to one embodiment of the present disclosure. In this example, each view 200 shows the fetus 202 and a fetal heart 208, as the orientation of the fetal heart 208 with respect to a pelvic inlet 206 may be indicative of the fetal presentation. For example, the view 200A represents the fetus 202A in a cephalic presentation with an according fetal heart orientation. The cephalic presentation means the head of the fetus 202 is in line with the pelvic inlet 206. Some embodiments of the present disclosure may determine the presentation of the fetus 202 by determining the location and orientation of the fetal heart 208. Additionally, the view 200B represents the fetus in a breech presentation. The breech presentation means the head of the fetus is pointed away from the pelvic inlet 206. Further, the view 200C represents an oblique presentation. The oblique presentation means the head of the fetus 202 is to the side of the pelvic inlet 206. Additionally, the view 200D represents a transverse presentation. The transverse presentation means the fetus is lying horizontally across the uterus. Knowing the presentation of the fetus may be useful in order to prepare for delivery, and/or provide therapy in the event of a breech, oblique, or transverse presentation.

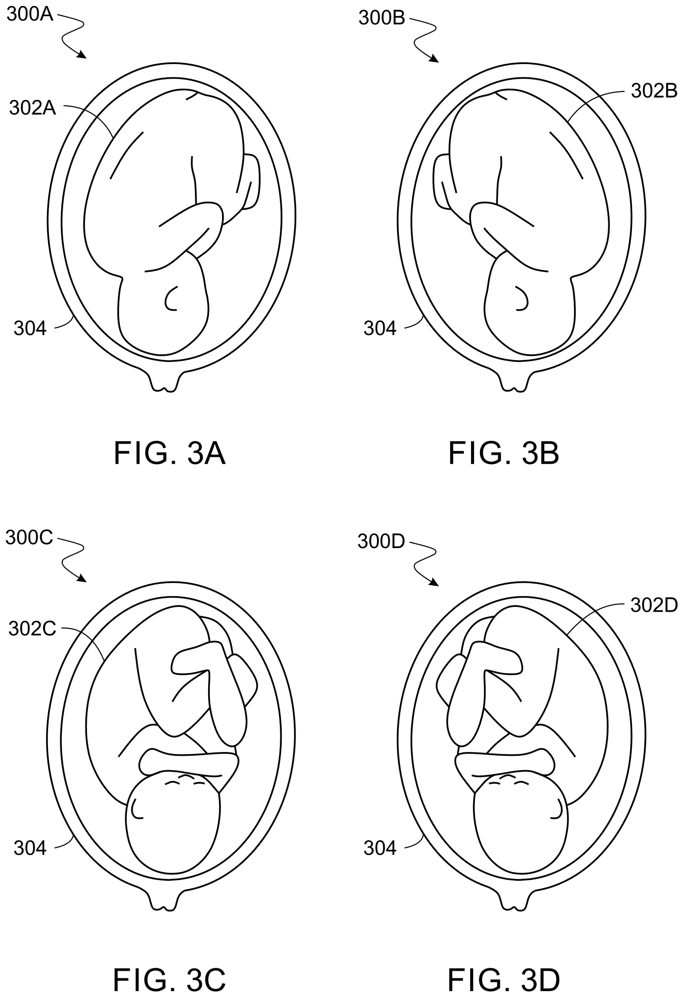

Figs. 3A, 3B, 3C, 3D are front views 300A, 300B, 300C, 300D (collectively referred to as views 300) of the respective positions of fetuses 302A, 302B, 302C, 302D (collectively referred to as fetuses 302) in a maternal abdomen 304, according to one embodiment of the present disclosure. The position, in contrast to the presentation, indicates the position of the fetal head as the fetal head enters the birth canal. In this example, each view 300 represents the fetus 302 in four different positions. More specifically, the view 300A represents the fetus 302A in a right occiput anterior position. The right occiput anterior position means the fetal occiput is facing the right side of the maternal abdomen 304. Additionally, the view 300B represents the fetus in a left occiput anterior position. The left occiput anterior position means the fetal occiput is facing the left side of the maternal abdomen 304. Further, the view 300C represents the fetus in a right occiput posterior position. The right occiput posterior position means the fetal occiput is facing the right side of the pregnant patient's back. Additionally, the view 300D represents the fetus in a left occiput posterior position. The left occiput posterior position means the fetal occiput is facing the left side of the pregnant patient's back. It is useful for a clinician to know the position of the fetus because the information may be useful in preparing for delivery, and/or providing therapy in the event the position is not occiput anterior.

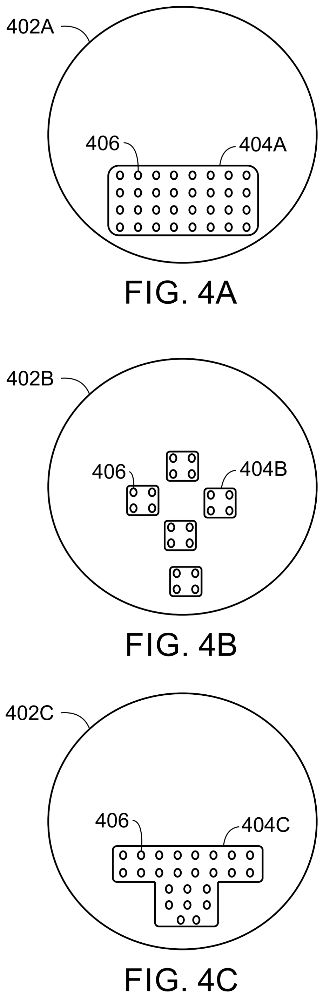

Fig. 4A is a front view of an example electrode grid patch 402A on a maternal abdomen 404, according to one embodiment of the present disclosure. The example electrode grid patch 402A includes grid electrodes 406, similar to the example electrode grid patch 102, described with respect to Fig. 1. In this example, the grid electrodes 406 are arranged in a four by eight (4 x 8) array. In these ways, the example electrode grid patch 302 may measure multi-channel EMG signals from the maternal abdomen with temporal and spatial information. The temporal and spatial information may refer to when and where a muscle is getting activated and how the action potential is travelling through the muscle fibers. Thus, each grid electrode 306 may conduct potentials from the muscles activating, with individual potentials indicating when and where.

In this arrangement, the example electrode grid patch 402A may lie: below the fetus earlier in labor; over the fetus as the fetus descends into and through the birth canal; and, above, as the fetus further descends. The differences in these relative positions may provide different EMG signal components as the fetus descends. By performing an analysis of the varying EMG signals as the fetus (and thus the fetal heart) changes location during labor, some embodiments of the present disclosure may compute fetal heart location, and thus, the position, presentation, and descent of the fetus. As stated previously, it may be possible to place the example electrode grid patch 402A in one position for monitoring throughout labor.

Fig. 4B is a front view of example electrode grid patches 402B on a maternal abdomen 404, according to one embodiment of the present disclosure. In this example, there are five electrode grid patches 402B, which are each similar to the electrode grid patch 102. The example electrode grid patches 402B include grid electrodes 406 and each is arranged in a 2 x 2 array. Having multiple electrode grid patches 402 may enable the clinician to place the electrode grid patches in multiple locations, which may provide a greater coverage volume of the maternal abdomen and the birth canal. In this way, the electrode grid patches 402 may detect signals across a greater physical range, potentially providing varying levels of magnitude of the fECG, mECG, and uterine activity, for example. The increase in the locations of the number of component signals (e.g., fECG) of the raw EMG, provide more data to analyze. Additionally, the differences in the detected signals across a greater volume of the abdomen may complement the data collected. Having such data may make it possible to improve the detection of component signals and also to determine the fetal heart location, position, presentation, and descent. As stated previously, it may be possible to place the example electrode grid patches 402B in one position for monitoring throughout labor.

Fig. 4C is a front view of example electrode grid patch 402C on the maternal abdomen 404, according to one embodiment of the present disclosure. While similar in location to the electrode grid patch 402A, the example electrode grid patch 402C includes multiple arrays in offset from neighboring arrays. This arrangement includes a 2 x 8 array, having an offset 2 x 3 array, having an offset 2 x 1 array. Further, the different array arrangements in the example electrode grid patch 402C may detect raw EMG signals in different locations than the grid electrodes 406 of the electrode grid patch 402A. Additionally, while the arrangement of grid electrodes 406 varies from the electrode grid patch 402A, the position of the electrode grid patch 402C is similar. As such, the electrode grid patches 402A, 402B, 402C may detect differences in the raw EMG signal strength as the fetus descends. As stated previously, detecting the varying strengths and patterns of the raw HD EMG signals as the fetus (and thus the fetal heart) changes location during labor, may enable some embodiments of the present disclosure to compute fetal heart location, and thus, the position, presentation, and descent of the fetus.

As stated previously, an electrode grid patch, such as the example electrode grid patches 402A, 402B, 402C (collectively referred to as example electrode grid patches 402) may provide a high-density EMG measurement system that can make it possible to detect fetal position, heart location, fetal presentation and fetal station. Additionally, the data provided by the example electrode grid patch 402 may be useful in computing where to position an ultrasound transducer to detect the fetal heart rate. According to some embodiments, it may be possible to perform these computations with reduced noise, in comparison to current techniques. Such embodiments may remove artefacts such as, movement, by comparing and/or combining signals across different pairs of grid electrodes 406, and using signal processing or extraction methods, as described in greater detail below.

Accordingly, the example electrode grid patch 402 may make it possible to detect fetal position, presentation, fetal heart location and fetal station. Further, such embodiments may reduce the practice of periodic palpation of the maternal abdomen, which may be uncomfortable for the mother, and time-consuming for the clinician. Further, such embodiments make it possible to continuously determine fetal descent, and present it graphically on a monitor, smartphone, or other display. Additionally, such embodiments may be useful at home (where pre-term labor is more likely) and for high-risk pregnancies. As stated previously, it may be possible to place the example electrode grid patch 402C in one position for monitoring throughout labor.

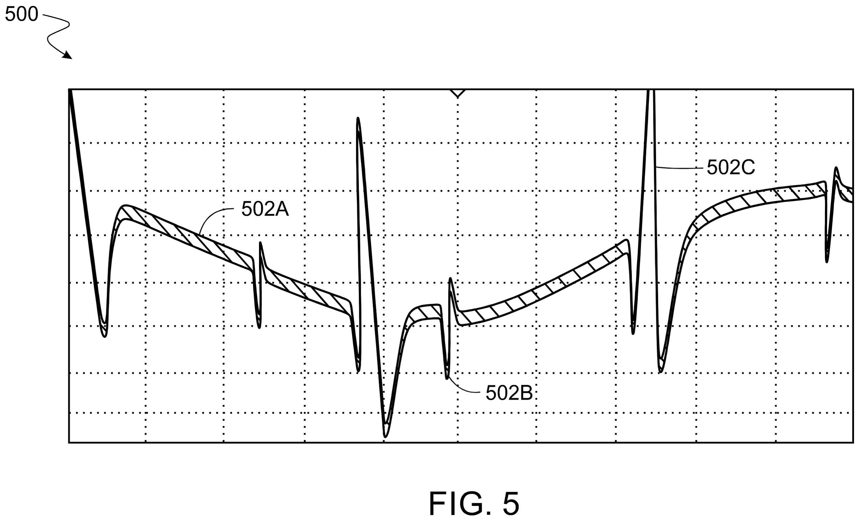

Fig. 5 is an example time-series view 500 of physiological monitoring from one (or a pair) of the electrodes of an example electrode grid patch, according to one embodiment of the present disclosure. As shown, the measured signals 502 is a collection of multiple specific signals and noise and artefacts such as noise due to mom's movements, electrical interference, etc. The specifically indicated signals 502A, 502B, 502C represent different elements of the detected signals. For example, the signal 502A indicates slow underlying behavior that may represent the uterine activity (contractions). However, the signal 502B may represent the fetal ECG (fECG), and the specifically indicated location (i.e., time) of the signal 502B may represent the peak of the Q, R, and S waves (QRS peak). The QRS waves may represent the ventricular depolarization. Similarly, the signal 502C may represent the maternal ECG (mECG), and the specifically indicated location, the QRS peak. It is useful to know the QRS peaks of the fECG and mECG, as these are indicators of heart muscle activity. Knowing when the heart muscle is contracting is useful because when (and thus, how frequently) the muscle is contracting indicates the heart rate.

Every electrode (or electrode-pair) has its own time-series of detected signals. At any given point in time, the signal from each electrode (or electrode pair) provides an EMG signal space map that is related to the regional activation of the muscles (or action potential space map) at that point in time. A collection of time-series data from the various electrodes (or electrodepairs) can provide a movie of the EMG signal map (which is related to the time evolution of the action potential map - i.e, how the action potential propagates along the muscle fibers). Using this spatiotemporal map, the properties of the muscles/muscle fibers such as the locations of the innervation zones and the propagation properties (direction and muscle fiber conduction velocity) can be determined, thereby making it possible to determine fetal presentation, fetal heart location and fetal station.

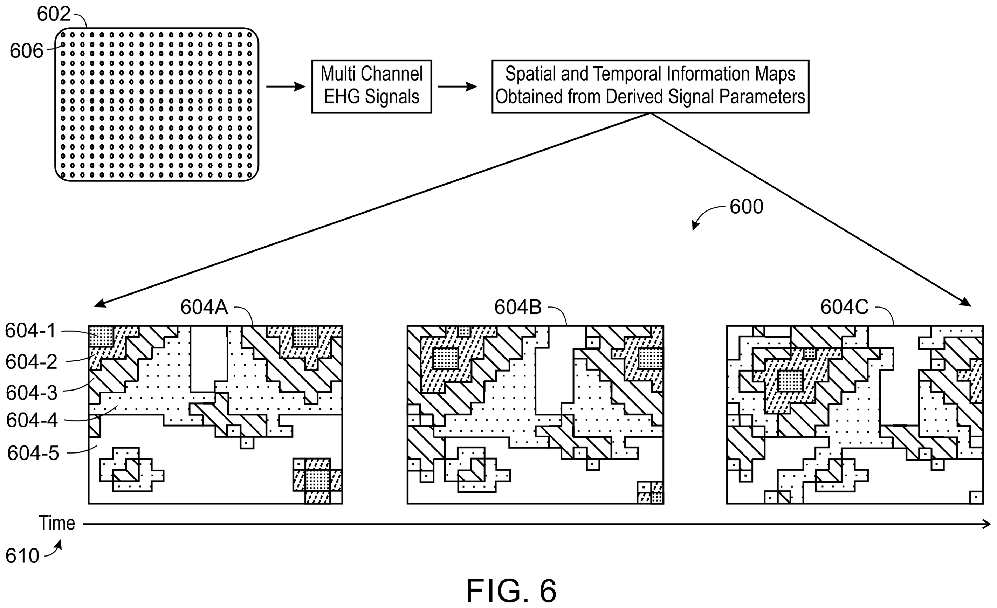

Fig. 6 is a diagram of an example electrode grid patch 602 and a timeline 600 of example mapping images 604A, 604B, 604C (collectively referred to as mapping images 604), according to one embodiment of the present disclosure. The example electrode grid patch 602 is similar to the electrode grid patches 102, 302 described with respect to Figs. 1 and 3. However, in this example, the electrode grid patch 602 includes a 20 x 16 array of grid electrodes 606. As indicated by the "Time" line 610, the mapping images 604 represent a view within the maternal abdomen during different stages of labor. The mapping images 604 may visualize the effect of the different action potentials at the measurement locations. More specifically, within each of the mapping images 604, the different areas 604-1, 604-2, 604-3, 604-4, 605-5 indicate different EMG signal levels, which are related to the locations and strength of the various component signals.

By analyzing mapping images, such as the example mapping images 604, some embodiments of the present disclosure, may compute the fetal heart location, and the presentation, position, and descent of the fetus through various stages of labor. Further, it may be possible to detect the raw EMG signals using the example electrode grid patch 602 positioned at a single location of the maternal abdomen, i.e., without moving the example electrode grid patch 602. According to some embodiments of the present disclosure, it may be possible to use a sequential image deep learning model to infer the fetal heart location, position, presentation, and descent based on mapping images, such as the example mapping images 604. More specifically, it may be possible to train a convolutional neural network to perform such classifications.

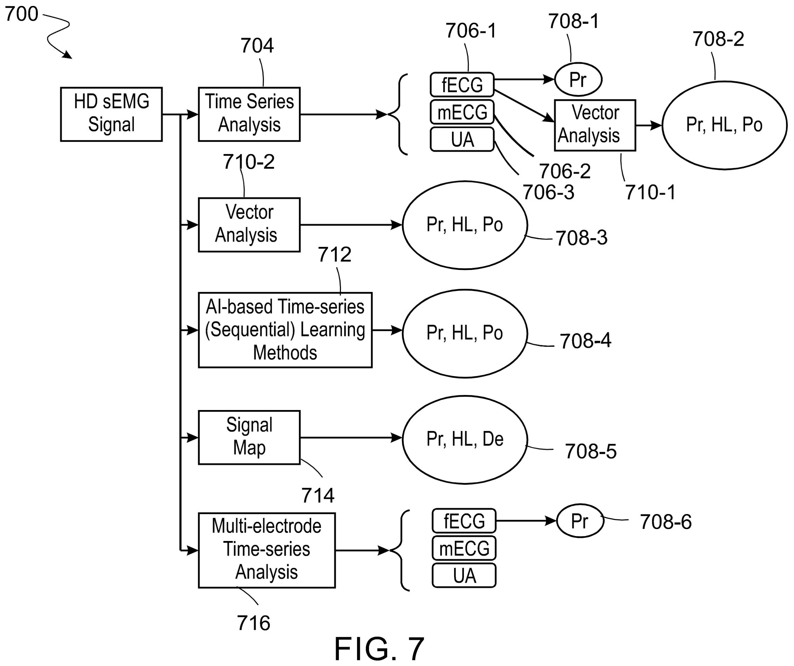

Fig. 7 is a data flow diagram of a process 700 for monitoring birth labor using an example electrode grid patch, according to one embodiment of the present disclosure. The process 700 uses a dataset of high-density EMG (HD EMG) signals 702 as inputs. The EMG signals 702 may be a collection of pre-processed EMG signals that some embodiments of the present disclosure may derive by perfoming filtering, wavelet processing and other signal processing techniques on the raw EMG signals. In these ways, such embodiments may remove noise, artefacts, and the like from the raw EMG signals.

According to some embodiments of the present disclosure, the EMG signals 702 may be input to a time series analysis 704. The time series analysis 704 may involve adaptive and non-adaptive filtering, wavelet analysis, wavelet coherence analysis, non-linear analysis, peak detection and correlation techniques. Additionally, the time series analysis 704 may involve pattern recognition, e.g., recognizing patterns of fetal and maternal ECG, uterine activity, and the like, in the EMG signals 702. In these ways, some embodiments of the present disclosure may extract the fetal ECG 706-1, maternal ECG 706-2, and uterine activity 706-3 from the EMG signals 702. Additionally, some embodiments of the present disclosure may use the fetal ECG signals 706-1 to determine fetal presentation by using pattern matching techniques. Accordingly, some embodiments of the present disclosure may compute the output 708-1, i.e., presentation (Pr). Also, such embodiments may perform vector analysis 710-1 on the fECG 706-1 to compute the output 708-2, i.e., presentation (Pr), fetal heart location (HL), and position (Po).

Additionally, the EMG signals 702 may be input to a vector analysis 710-2. The vector analysis 710-2 may involve an analysis in which EMG signal from each electrode is represented in amplitude and direction format. Vector analysis may be used in interpreting the multidimensional nature of the EMG signals, typically derived from multiple electrodes, as in example electrode patch 602, to study muscle activity. By performing spatial and temporal analysis of EMG vector components, such embodiments may derive the variation of different groups of muscle activations over time. Preprocessed EMG signals may be used for vector analysis. Vector analysis between different electrodes may be done using different metrics and methods such as Riemannian metric method, Correlation coefficient metric, Kullback-Leibler Divergence metric, wavelet coherence method, principal component analysis etc. These vector analysis and additional computations may be used for identifying fetal heart location, position and presentation. In these ways, the vector analysis 710-2 may produce the output 708-3, i.e., Pr, HL, and Po.

Further, the EMG signals 702 may be input to artificial intelligence (AI) based time-series learning methods 712. The AI-based time-series learning methods 712 may involve training a recurrent neural network (RNN), e.g., a long short-term memory (LSTM) model and bidirectional LSTM (BiLSTM) model to infer the output 708-4, i.e., Pr, HL, and Po. The BiLSTM model may be trained with supervised learning to perform classifications with respect to presentation and position based on a dataset of historical processed EMG signals. Accordingly, the trained BiLSTM may infer the output 708-4 using the EMG signals 702 for a maternal patient and fetus(es) in real-time.

Additionally, some embodiments of the present disclosure may input the EMG signals 702 to a signal map process 714. The signal map process 714 may generate mapping images, such as the mapping images 604 described with respect to Fig. 6, by using mapping points. Such embodiments may derive the mapping points from the EMG signals 702. In some embodiments, deriving the mapping points may involve using mean square values, mean values, and/or any of the linear and non-linear parameters derived from the EMG signals 702. Additionally, the signal map process 714 may using deep learning models, such as a convolutional neural network (CNN) to infer the output 708-5, i.e., Pr, HL, and De based on the mapping images. The CNN may be trained using supervised learning and a training dataset of mapping images generated from historical EMG signal data. As stated previously, the mapping images 604 may visualize the effect of all the different action potentials at multiple measurement locations. Accordingly, a CNN may deconvolute the mapping images. In this way, it may be possible for the CNN to determine which signal is coming from where, how the signals are travelling (e.g., throughout labor), and the like. Further, based on this deconvolution or other types of analysis or other sequential image DL and classification algorithms, some embodiments of the present disclosure may determine the output 708-5.

More specifically, some embodiments of the present disclosure may use the CNN model to compute presentation and position classifications. For example, the CNN model may include one or more artificial intelligence algorithms to process the input mapping images 604 to identify fetal heart location, position, presentation, and station (e.g., descent) within the mapping images 604. Additionally, the CNN model may use numerous weights and biases, activation functions, loss functions, gradient descent algorithms, and instructions classifying maping images 604 in these ways. Further, the deep learning models may include trained and/or untrained neural networks and may further include training routines, or parameters (e.g., weights and biases), associated with one or more neural network models stored therein. Additionally, the deep learning models may include image recognition algorithms, shape or edge detection algorithms, and the like. Further, such models may evaluate the mapping images 604 as acquired in real-time. Additionally or alternatively, the deep learning models may evaluate the mapping images 604 offline, e.g., not in real-time.

Additionally, some embodiments of the present disclosure may input the EMG signals 702 to a multi-electrode time-series analysis 716. The multi-electrode time-series analysis 716 may be similar to the time series analysis 704. However, the multi-electrode time-series analysis 716 may involve combining and/or comparing signals from different grid electrodes, with respect to multiple grid electrode pairs. Further, Combining signals across different electrodes or different pairs of electrodes, and using different signal processing methods (such as averaging) may generate relatively high quality and relatively low noise fECG, mECG and UA. Additionally, combining signals across different electrodes or different pairs of electrodes, and using different signal processing methods (such as averaging) may improve the quality, and reduce the noise, of the fECG, mECG and UA. In this way, such embodiments may generate the output 708-6, i.e., presentation (Pr).

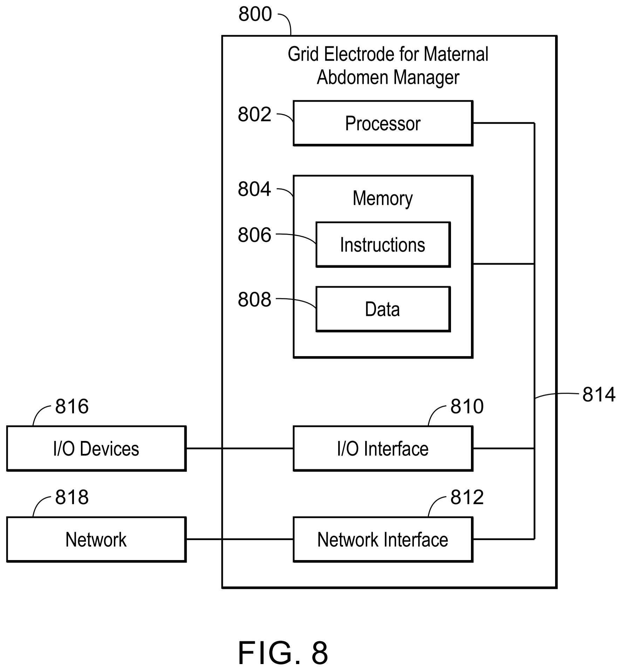

Fig. 8 is a diagram of an example maternal abdomen electrode grid patch manager 800, according to one embodiment of the present disclosure. The example maternal abdomen electrode grid patch manager 800 may generate raw and processed EMG signals for each electrode; extract fECG, mECG and UA signals; generate mapping images; and determine fetal heart location, presentation, position, and fetal heart station, as described with respect to Figs. 1, 4A, 4B, 4C, and 5-7. In this example, the example maternal abdomen electrode grid patch manager 800includes a processor 802, memory 804, input-output (I/O) interface 810, and network interface 812, which may be connected by an interconnect 814. The processor 802 may be a computer processing circuit (e.g., a central processing unit (CPU)) that retrieves and executes programming instructions 806 stored in the memory 804 to perform the functionality described herein. The interconnect 814 may move data, such as programming instructions, between the processor 802, memory 804, I/O interface 810, and network interface 812. The interconnect 814 may include one or more buses.

The memory 804 may be a computer memory or storage device, including volatile memory, such as a random access memory (RAM) device (e.g., static RAM, dynamic RAM, and the like), non-volatile memory, such as a hard disk drive, solid state device (SSD), removable memory cards, optical storage, flash memory devices, and the like. In some examples, the memory 804 may include volatile and non-volatile memory devices. Further, the memory 804 may store instructions 806, described above, and data 808. The data 808 may include the EMG signals detected by the grid electrode patch for maternal abdomen, and the times when the EMG signals are detected. Additionally, the data 808 may include thresholds for determining the station.

Additionally, the example maternal abdomen electrode grid patch manager 800may be in electronic communication with I/O devices 816 through the I/O interface 810, and with a network 818 through the network interface 812. The I/O devices 816 may capture inputs and provide outputs as described herein. The network 818 may be an electronic communication network, such as a local area network, wide area network, and the like, for processing communications between the example maternal abdomen electrode grid patch manager 800and the deep learning models and AI models described herein. In some examples, the network 818 may be wired, wireless (e.g., wi-fi, Bluetooth, or cellular), or some other computer communication network.

In some embodiments, the example maternal abdomen electrode grid patch manager 800may be a server computer or similar device without a user interface but which receives requests from other computer systems having one or more user interfaces. Further, in some embodiments, the example maternal abdomen electrode grid patch manager 800may be a portable computer, laptop, tablet computer, pocket computer, telephone, smart phone, or the like.

An example method includes determining fetal parameters using at least one electrode grid patch that is disposed on a maternal abdomen of a pregnant patient, by receiving an abdominal electromyogram (EMG) signal from the at least one electrode grid patch. Additionally, the at least one electrode grid patch includes multiple electrodes that are disposed in at least one array. Further, the method includes determining fetal parameters by extracting, from the EMG signal, a fetal electrocardiogram (ECG) signal of a fetus of the pregnant patient. Additionally, the method includes determining fetal parameters by determining for the fetus, based on the EMG signal, a fetal presentation. Further, the method includes providing for display on a display device, the fetal presentation.

In another example, the method includes extracting, from the EMG signal: a maternal ECG signal and a uterine activity signal. Additionally, the method includes determining for the fetus, based on the EMG signal: a fetal heart location, a fetal position, and a fetal station.

In another example, determining the fetal presentation, the fetal position and the fetal heart location includes performing a vectorial analysis on the EMG signal.

In another example, determining the fetal presentation, the fetal position, and the fetal heart location includes performing a vectorial analysis on the fetal ECG signal.

In another example, the method includes generating amplitude and direction of the fetal ECG signal by performing vectorial analysis.

In another example, the method includes performing vectorial analysis on the EMG signal. Additionally, performing the vectorial analysis includes generating multiple vectors corresponding to the electrodes. Further, each of the vectors includes an amplitude and a direction for the components of the EMG signal. Additionally, performing the vectorial analysis includes determining multiple resultant vectors corresponding to the vectors. Further, each of the resultant vectors results from a comparison parameter between the amplitude and the direction for each of the vectors for each of the electrodes. Additionally, performing the vectorial analysis includes determining the fetal heart location, the fetal presentation and the fetal position based on the resultant vectors.

In another example, determining the fetal presentation includes performing a pattern recognition analysis of the fetal ECG signal

In another example, determining the fetal presentation, the fetal heart location, and the fetal position includes performing a classification on a sequential time-series analysis of the EMG signal.

In another example, determining the fetal station, the fetal presentation, the fetal position, and the fetal heart location include generating multiple sequential input mapping images of the maternal abdomen based on the EMG signal. Additionally, determining the station, presentation, and heart location include using a sequential deep learning model that is trained to classify the fetal station, the fetal presentation, the fetal position, and fetal heart location, based on the plurality of input mapping images.

In another example, determining the fetal ECG signal, maternal ECG and uterine activity signal include increasing signal quality and reducing signal noise by performing multi-electrode analysis and signal processing techniques on the EMG signal.

In another example, the method includes extracting, from the EMG signal an additional fetal ECG signal for an additional fetus, and determining for the additional fetus, based on the EMG signal, an additional fetal presentation, an additional fetal heart location, an additional fetal position, and an additional fetal station.

In another example, extracting the additional fetal ECG signal includes separating the fetal ECG signal from the additional fetal ECG signal by using source separation methods.

An example system includes at least one electrode grid patch, a processing device, and a memory. The electrode grid patch includes multiple electrodes disposed in at least one array. Additionally, the electrode grid patch is configured for placement on a maternal abdomen of a pregnant patient, and to conduct potentials of the pregnant patient, and a fetus of the pregnant patient. The memory includes instructions that are executable by the processing device to receive an EMG signal from at least one electrode grid patch, the at least one electrode grid patch including multiple electrodes that are disposed in at least one array. Further, the instructions are executable by the processing device to determine for the fetus, based on the EMG signal, a fetal position. Additionally, the instructions are executable by the processing device to provide for display on a display device, the fetal position.

In another example, the instructions are executable by the processing device to extract, from the EMG signal: a maternal ECG signal and a uterine activity signal. Further, the instructions are executable by the processing device to determine for the fetus, based on the EMG signal: a fetal heart location, a fetal presentation, and a fetal station.

In another example, the fetal position, the fetal presentation, and the fetal heart location are determined by performing a vectorial analysis on the fetal ECG signal, the maternal ECG signal, and the uterine activity signal.

In another example, the fetal position, the fetal presentation, and the fetal heart location are determined by performing a vectorial analysis on the EMG signal.

In another example, the instructions are executable by the processing device to perform the vectorial analysis by generating multiple vectors corresponding to the electrodes. Further, each of the vectors includes an amplitude and a direction for multiple components of the EMG signal. Additionally, the vectorial analysis is performed by determining multiple resultant vectors corresponding to the vectors. Further, each of the resultant vectors results from a comparison parameter between the amplitude and the direction for each of the vectors for each pair of the electrodes. Additionally, the vectorial analysis is performed by determining the fetal heart location, the fetal presentation and the fetal position based on the resultant vectors.

In another example, the instructions are executable by the processing device to determine a fetal engagement of the fetus based on the fetal ECG signal, the maternal ECG signal, and the uterine activity signal.

In another example, determining the fetal presentation, the fetal heart location, and the fetal engagement include using a sequential artificial intelligence model that is trained to infer the fetal presentation, the fetal heart location, and the fetal engagement, at a plurality of times, based on the EMG signal.

In another example, the instructions are executable by the processing device to increase signal quality and reduce signal noise of the fetal ECG signal, the maternal ECG signal, and the uterine activity signal by performing multi-electrode analysis and signal processing techniques on the EMG signal.

An example method includes determining fetal parameters using at least one electrode grid patch for a maternal abdomen that is disposed on a maternal abdomen of a pregnant patient, by receiving an abdominal electromyogram (EMG) signal from the at least one electrode grid patch. Additionally, the at least one electrode grid patch includes multiple electrodes that are disposed in at least one array. Further, the method includes determining fetal parameters by determining for a fetus of the pregnant patient, based on the EMG signal, a fetal station. Further, the method includes providing for display on a display device, the fetal station.

As used herein, the term, mechanism, can encompass hardware, software, firmware, or any suitable combination thereof. In some embodiments, any suitable computer readable media can be used for storing instructions for performing functions and/or processes described herein. For example, in some embodiments, computer readable media can be transitory or non-transitory. For example, non-transitory computer readable media can include media such as magnetic media (such as hard disks, floppy disks, etc.), optical media (such as compact discs, digital video discs, Blu-ray discs, etc.), semiconductor media (such as RAM, Flash memory, electrically programmable read only memory (EPROM), electrically erasable programmable read only memory (EEPROM), etc.), any suitable media that is not fleeting or devoid of any semblance of permanence during transmission, and/or any suitable tangible media. As another example, transitory computer readable media can include signals on networks, in wires, conductors, optical fibers, circuits, or any suitable media that is fleeting and devoid of any semblance of permanence during transmission, and/or any suitable intangible media.

As used herein, the term, mechanism, can encompass hardware, software, firmware, or any suitable combination thereof. In some embodiments, any suitable computer readable media can be used for storing instructions for performing functions and/or processes described herein. For example, in some embodiments, computer readable media can be transitory or non-transitory. For example, non-transitory computer readable media can include media such as magnetic media (such as hard disks, floppy disks, etc.), optical media (such as compact discs, digital video discs, Blu-ray discs, etc.), semiconductor media (such as RAM, Flash memory, electrically programmable read only memory (EPROM), electrically erasable programmable read only memory (EEPROM), etc.), any suitable media that is not fleeting or devoid of any semblance of permanence during transmission, and/or any suitable tangible media. As another example, transitory computer readable media can include signals on networks, in wires, conductors, optical fibers, circuits, or any suitable media that is fleeting and devoid of any semblance of permanence during transmission, and/or any suitable intangible media.

This written description uses examples to disclose the invention, including the best mode, and also to enable any person skilled in the art to make and use the invention. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

## Claims

1. A method, comprising:
determining fetal parameters using at least one electrode grid patch that is disposed on a maternal abdomen of a pregnant patient, by:
receiving an abdominal electromyogram (EMG) signal from the at least one electrode grid patch, the at least one electrode grid patch comprising a plurality of electrodes that are disposed in at least one array; and
determining for a fetus of the pregnant patient, based on the EMG signal, a fetal presentation; and
providing for display on a display device, the fetal presentation.

2. The method of claim 1, comprising:
extracting, from the EMG signal:
a fetal electrocardiogram (ECG) signal of the fetus;
a maternal ECG signal; and
a uterine activity signal;
determining for the fetus, based on the EMG signal:
a fetal heart location;
a fetal position; and
a fetal station; and
providing for display on the display device:
the fetal ECG signal;
the maternal ECG signal;
the uterine activity signal;
the fetal heart location;
the fetal position; and
the fetal station.

3. The method of claim 2, determining the fetal presentation, the fetal position and the fetal heart location comprising performing a vectorial analysis on the EMG signal.

4. The method of claim 2, determining the fetal presentation, the fetal position, and the fetal heart location comprising performing a vectorial analysis on the fetal ECG signal.

5. The method of claim 4, comprising generating amplitude and direction of the fetal ECG signal by performing a vectorial analysis.

6. The method of claim 3, comprising performing vectorial analysis on the EMG signal, performing the vectorial analysis comprising:
generating a plurality of vectors corresponding to the plurality of electrodes, each of the plurality of vectors comprising an amplitude and a direction for a plurality of components of the EMG signal;
determining a plurality of resultant vectors corresponding to the plurality of vectors, each of the resultant vectors resulting from a comparison parameter between the amplitude and the direction for each of the vectors for each of the electrodes; and
determining the fetal heart location, the fetal presentation and the fetal position based on the plurality of resultant vectors.

7. The method of claim 1, determining the fetal presentation comprising performing a pattern recognition analysis of the fetal ECG signal.

8. The method of claim 2, determining the fetal presentation, the fetal heart location, and the fetal position comprising performing a classification on a sequential time-series analysis of the EMG signal.

9. The method of claim 2, determining the fetal station, the fetal presentation, the fetal position, and the fetal heart location comprising:
generating a plurality of sequential input mapping images of the maternal abdomen based on the EMG signal;
determining the fetal station, the fetal presentation, the fetal position, and the fetal heart location by using a sequential deep learning model that is trained to classify the fetal station, the fetal presentation, the fetal position, and the fetal heart location based on the plurality of sequential input mapping images.

10. The method of claim 2, determining the fetal ECG signal, the maternal ECG signal, and the uterine activity signal comprising increasing signal quality and reducing signal noise by performing multi-electrode analysis and signal processing techniques on the EMG signal.

11. The method of claim 2, comprising:
extracting, from the EMG signal, an additional fetal ECG signal for an additional fetus; and
determining for the additional fetus, based on the EMG signal:
an additional fetal presentation;
an additional fetal heart location;
an additional fetal position; and
an additional fetal station.

12. The method of claim 2, extracting the additional fetal ECG signal comprising separating the fetal ECG signal from the additional fetal ECG signal by using source separation methods.
